# EUROPEAN PATENT APPLICATION

(11) **EP 3 449 965 A1**
(43) Date of publication of application: **06.03.2019**
(21) Application number: 17189490.0
(22) Date of filing: 05.09.2017
(51) Int. Cl.: A61M 25/00, A61M 25/01

(54) **STEERABLE CATHETER WITH PORTIONS OF DIFFERENT STIFFNESS**

(71) Applicant: ETH Zurich, 8092 Zurich (CH)
(72) Inventor: The designation of the inventor has not yet been filed

(57) **Abstract**

A steerable catheter (10) comprises portions (100, 105) of different stiffness, having a proximal end (11), a distal end, a lumen extending there between, at least one magnet (20) at the proximal end (11), at least a magnetic field to influence the proximal end (11), wherein portions (100, 105) of the steerable catheter (10) can be of different stiffness. The portions (100, 105) of different stiffness are in fact portions of variable stiffness based on a phase change between a liquid phase and a solid phase of a liquid-solid material in a at least partly double walled catheter providing a cavity for this liquid-solid material and having additional control elements configured to influence the phase of the liquid-solid material.

## Description

### TECHNICAL FIELD

The present invention relates to a steerable catheter with portions of different stiffness, having: a proximal end, a distal end, a lumen extending there between, at least one magnet at the proximal end, at least a magnetic field to influence the proximal end, wherein portions of the steerable catheter can be of different stiffness.

### PRIOR ART

Such a catheter having the features mentioned above is known from WO 01/17600 A1.

Another prior art catheter is US 2008/009831 A, wherein the catheter assembly includes an elongate shaft comprising a thermoplastic polymer such as a thermoplastic shape memory polymer having a pre-selected glass transition temperature (Tg) and a means for heating the thermoplastic polymer, wherein the thermoplastic polymer is in a rubbery state at temperatures above the glass transition temperature and is in a glassy state at temperatures below the glass transition temperature. The elongate shaft may be selectively heated and cooled to provide sufficient flexibility and retention during a medical procedure. [0033] of US 2008/009831 A names a number of possibilities how to heat and cool the shape memory polymer.

Remote magnetic navigation of catheters is a technique to used to perform radiofrequencey ablation of heart tissue in order to treat - inter alia - cardiac arrhythmias. The flexible magnetic catheter as mentioned above can be, in some cases, not dexterous enough to navigate the complex and patient-specific anatomy of the heart.

### SUMMARY OF THE INVENTION

Based on the above prior art it is an object of the invention to provide a steerable catheter with portions of different stiffness having improved steering functions.

The present invention provides a steerable catheter with portions of different stiffness, having: a proximal end, a distal end, a lumen extending there between, at least one magnet at the distal end, at least a magnetic field to influence the distal end, wherein portions of the steerable catheter can be of different stiffness, which is characterized in that the portions of different stiffness are in fact portions of variable stiffness based on a phase change between a liquid phase and a solid phase of a liquid-solid material in a at least partly double walled catheter providing a cavity for this liquid-solid material and having additional control elements configured to influence the phase of the liquid-solid material.

In one embodiment the liquid-solid phase transition is based on heating or cooling off the liquid-solid material. The liquid-solid phase transition can be based on heating or cooling off the liquid-solid material. The control elements can comprise a resistive wire heating coil.

The control elements can also comprise direct heating through electrodes provided across the catheter, either between two positions in the longitudinal direction axis or radially between the inner side of the outer flexible tube and the outer side of the inner flexible tube.

The liquid-solid phase transition can also be based on heating or cooling off the liquid-solid material through peltier elements, provided in the walls, preferably in the inner walls of the cavity for the liquid-solid material.

Further embodiments of the invention are laid down in the dependent claims.

During an ablation procedure multiple catheters are used and remote magnetic navigation can only control a single catheter. With the present catheter, it is possible to first move a reference catheter to a target position and subsequently move an ablation catheter to the ablation spot.

The magnet in the catheter can also be an electromagnet.

### BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,
- Fig. 1: shows a schematic perspective overview of a catheter according to an embodiment of the invention;
- Fig. 2: shows a schematic function overview of the catheter of Fig. 1;
- Fig. 3: shows a schematic cross-section side view of a variable stiffness section according to a first embodiment of the invention;
- Fig. 4: shows a schematic cross-section side view of a variable stiffness section according to a second embodiment of the invention;
- Fig. 5: shows a schematic cross-section side view of a variable stiffness section according to a third embodiment of the invention;
- Fig. 6A: shows a schematic cross-section of a catheter according to an embodiment of the invention;
- Fig. 6B: shows a schematic cross-section of a further catheter according to an embodiment of the invention;
- Fig. 6C: shows a schematic cross-section of a further catheter according to an embodiment of the invention;
- Fig. 7: shows a diagram of a control system for a catheter according to an embodiment of the invention;
- Fig. 8: shows a diagram of resistance against time of an example of a resistive wire resistance variation over time during cooling;
- Fig. 9: shows a schematic view of the effect of a heated heat source for a small or high energy injection; and
- Fig. 10: shows a schematic cross-section side view of a variable stiffness section according to a further embodiment of the invention.

### DESCRIPTION OF PREFERRED EMBODIMENTS

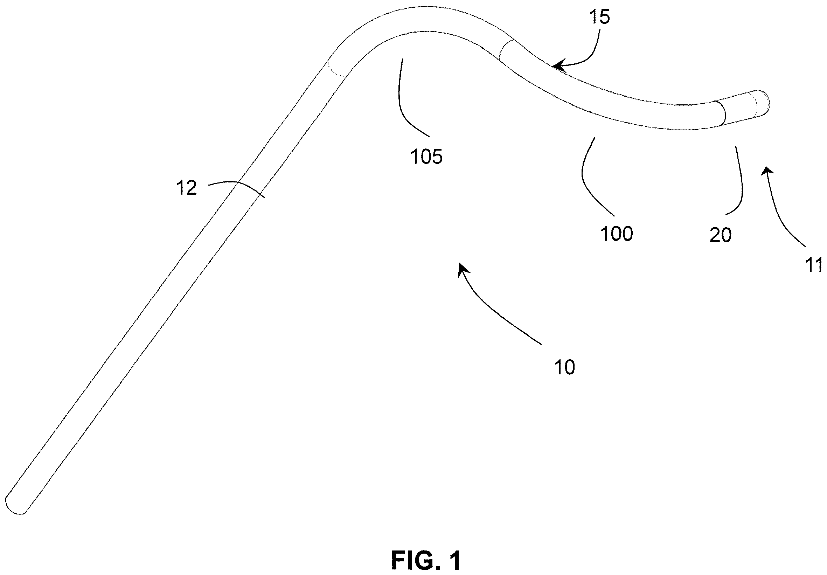

Fig. 1 shows a schematic perspective overview of a catheter 10 according to an embodiment of the invention. The catheter 10 comprises a tip 11 and a shaft 12. Near the tip is provided a magnet 20 which is influenced by an exterior magnetic field controlled to orient the tip 11 of the catheter 10 into a predetermined direction. Behind the tip 11 and in front of the shaft 12 is provided a variable stiffness region 15. Here, this region 15 comprises a first variable stiffness section 100 and a second variable stiffness section 105. The magnet 20 is a permanent magnet.

In other embodiments, there might be only one such variable stiffness section 100 or more than two. Such variable stiffness sections 100, 105 can be separated by shaft sections. It is also possible to provide the magnet 20 as a plurality of magnet parts and extending the variable stiffness section 100 longitudinally into magnet area.

On the other side, the tip 11 can comprise a functional element like a radiofrequeny ablation unit as known from the prior art.

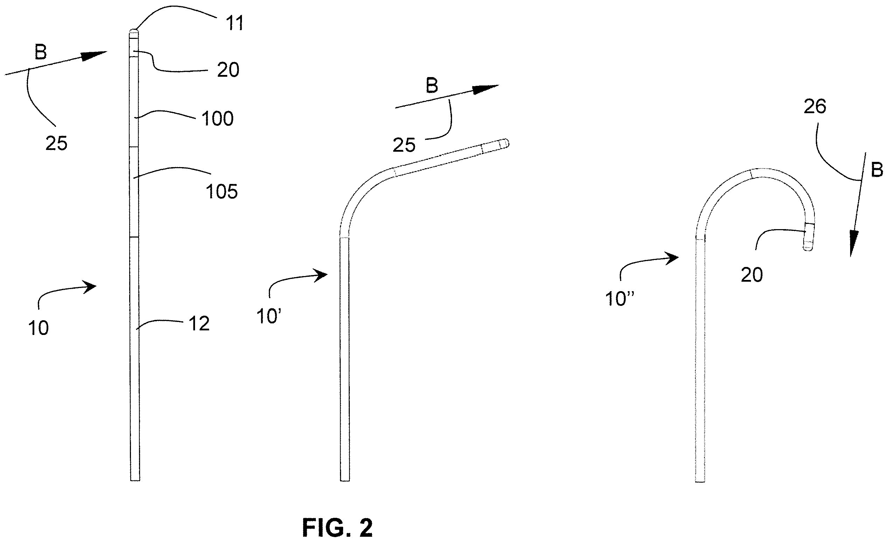

Fig. 2 shows a schematic function overview of the catheter 10 of Fig. 1 in three bending states in view of an applied magnetic field in the direction of vector B. Therefore, Fig. 2 is considered to be one single drawing, since the same catheter 10 is shown in three positions following the adjustment of the variable stiffness sections 100 and 105 and different orientations of the magnetic field according to the representing arrow 25 as well as 26.

Identical reference numerals are used throughout all drawings for identical features.

On the left side of Fig. 2 is shown a straight catheter 10. Shaft 12 is providing the vertical orientation which usually can be taken as reference since this part is located - when the catheter 1 is used e.g. for the above mentioned ablation- somewhere between the entering place of the catheter 10 into a vein of an patient and the heart region. The two variable stiffness sections 100 and 105 are in straight continuation of the shaft 12 and are followed by the magnet 20 and the tip 11. Both variable stiffness sections 100 and 105 are in the rigid state. It is the property of the variable stiffness sections 100 and 105 that they can bend freely as shown in Fig. 1 and e.g. follow the bends of a blood vessel, or that they can be in a rigid state. It will be seen that being in the rigid state is here not connected with the property "being straightly aligned". Here, in the representation on the left, the two variable stiffness sections 100 and 105 were already rigid, when the magnetic field with the direction of arrow 25 was applied. Although the magnet 20 is initially not aligned with the magnetic field vector 25, it is not aligning itself to the direction of vector 25, since the rigid variable stiffness sections 100 and 105 maintain the immediately adjacent magnet 20, immediately adjacent to variable stiffness sections 100, in direct prolongation of the proximal part 101 of the variable stiffness section 100.

The representation in the middle of Fig. 2 shows what happens to the catheter 10' when the second variable stiffness section 105 is actively switched from the rigid state to the flexible state while maintaining the first variable stiffness section 100 in its rigid state. Then the magnet 20 can align itself to the unchanged direction 25 of the magnetic field, while the first variable stiffness section 100 remains with its rigid state.

Finally, the representation on the right side shows a further evaluation of the situation of Fig. 2 with the catheter 10". While the direction of the magnetic field is maintained in the direction 25, the second variable stiffness section 105 is switched back to the rigid state and the first variable stiffness section 100 is switched from the rigid state to the flexible state. Then the control system is adjusted and the direction of the magnetic field is switched from the direction represented by the arrow 25 to the direction 26. Then it is possible for the first variable stiffness section 100 to bend allowing the magnet 20 to become aligned with the new direction 26 of the magnetic field.

The three representations of the catheter in three subsequent adjustments can be summarized in a table as:

| No. | Image | first section | second section | magnetic field | development |
|---|---|---|---|---|---|
| 1 | catheter position 10 | rigid | rigid | none | none |
| 2 | catheter position 10 | rigid | rigid | direction 25 | none |
| 3 | catheter position 10' | rigid | flexible | remain | bending of 2nd portion |
| 4 | catheter position 10' | rigid | rigid | remain | none |
| 5 | catheter position 10' | rigid | rigid | direction 26 | none |
| 6 | catheter position 10" | flexible | rigid | remain | bending of 1 st portion |

"Remain" has the meaning that the direction of the magnetic field of the previous step is maintained. Then, it is noted that the steps 2 and 3 can be rotated, since, if there is no magnetic field ("remain" = no magnetic field remains), the second section can become flexible and then the magnetic field is activated with the direction 25 providing the bending of the second portion at this point in time. Rotating the steps 5 and 6 will result in a small bending of 1st portion to reduce a none perfect alignment between magnet and magnetic field during step 3.

| No. | Image | first section | second section | magnetic field | development |
|---|---|---|---|---|---|
| 1 | catheter position 10 | rigid | rigid | None | none |
| 2 | catheter position 10 | rigid | flexible | Remain | none |
| 3 | catheter position 10' | rigid | rigid | direction 25 | bending of 2nd portion |
| 4 | catheter position 10' | rigid | rigid | Remain | none |
| 5 | Not shown in the drawings | flexible | rigid | Remain | Small bending of 1 st portion to compensate none perfect alignment of 2^{nd} portion |
| 6 | catheter position 10" | flexible | rigid | direction 26 | bending of 1 st portion |

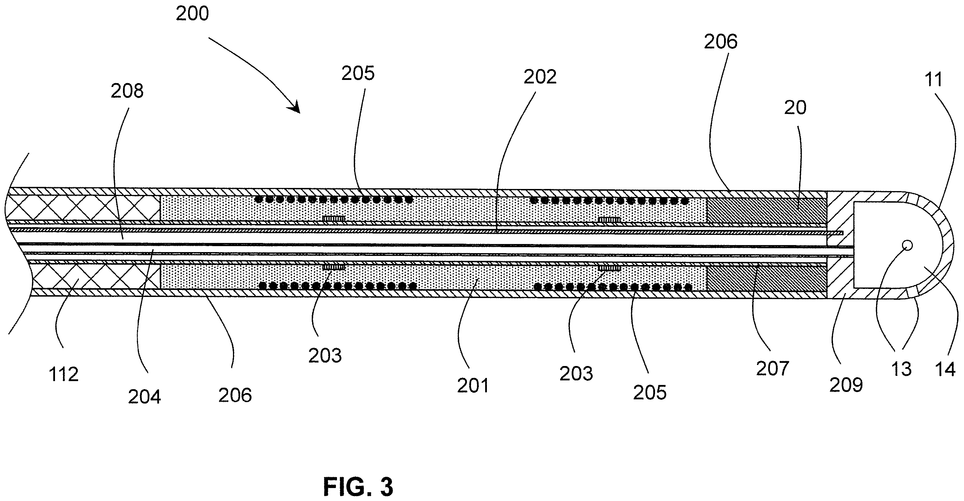

Fig. 3 shows a schematic cross-section side view of a variable stiffness section 200 according to a first embodiment of the invention using a resistive wire heater. The catheter 10 comprises an outer flexible tube encapsulation 206 and an inner flexible tube encapsulation 207. The outer flexible tube encapsulation 206 is thicker, especially two to three times thicker, than the inner flexible tube encapsulation 207. In between these two flexible films 206 and 207 is provided in the distal portion shown a shaft material 112 followed by a lumen filled with the variable stiffness material 201. Adjacent to this lumen is the magnet 20 which ends at the rear wall 209 of the tip 11. It is also possible to provide the lumen for the variable stiffness material 201 with direct walls between the two flexible films 206 and 207 to create the section 100 or 105.

Within the lumen for the variable stiffness material 201 are provided two helical portions of resistive wires 205 which are provided as two separate coils separated in the longitudinal direction of the catheter at the inner side of the outer flexible film 206. The resistive wires 205 are connected with the exterior, the power supply and the control unit delivering the heating current via two wires which are not represented in Fig. 3. They are usually provided in the lumen for the shaft material 112.

Within the lumen for the variable stiffness material 201 are provided several temperature sensors 203 which are provided at two separate positions separated in the longitudinal direction of the catheter at the outer side of the inner flexible film 207. Thus they take the temperature of the variable stiffness material 201. The temperature sensors 203 are connected with the exterior and the control unit receiving the measurements signals via wires which are not represented in Fig. 3. They are usually provided in the lumen for the shaft material 112.

Inside the inner flexible film 207 is the inner lumen 208, extending from the outside through the entire catheter until the rear wall 209 of the tip 11. Inside this lumen 208 is provided wires 202 to the tip which are lodged in the rear wall 209. The wires 202 can be connected in the tip to an ablation RF transmitter or electrodes to record cardiac activity.. Inside this lumen 208 is also provided a lumen 204 for a cooling fluid. This lumen 204 is built by a further pipe inside lumen 208 and extending through the rear wall 209. Then cooling fluid can be provided from the outside through the lumen 204 to cool all elements outside from the flexible wall 207 and exits later on the lumen 208 into the hollow tip cavity 14 and exits through one of the irrigation holes 13 In the absence of a functional tip 11, this inner lumen 204 can be used as tool channel to introduce any interventional tool.

In this first embodiment the conducting wire 205 was wound around the variable stiffness material 201 in its rigid state and a current circulating through the copper wire 205 heats the variable stiffness material 201. The resistive wire 205 is split in two different parts providing the possibilities to activate none, the front, the back or both resistive wires 205. It is also possible to use more, i.e. a plurality of resistive wires.

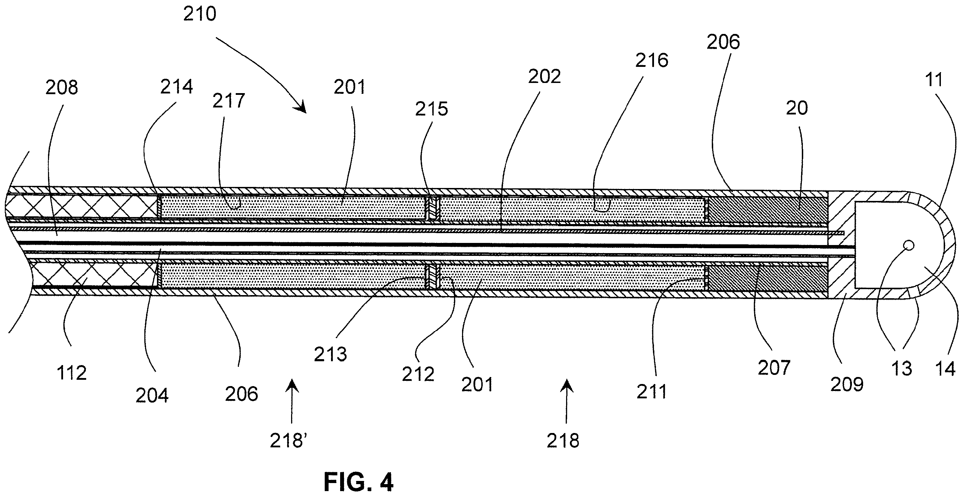

Fig. 4 shows a schematic cross-section side view of a variable stiffness section 210 according to a second embodiment of the invention using direct heating. Here, the current is flowing directly inside the variable stiffness material 201 via four electrodes 211, 212, 213 and 214. All features identical to features of the embodiment of Fig. 3 have the same reference numerals and are not mentioned again here.

The variable stiffness section 210 comprises two separate variable stiffness section 218 and 218'. They are separated by a thermal insulation torus or ring 215. On both sides of the thermal insulation torus 215 between the outer flexible material 207 and the inner flexible material 206 are provided one electrode 212 and 213, respectively. Near the magnet is provided the ring-shaped electrode 211 and on the shaft material 112 is provided the ring-shaped electrode 214. The electrode 211 is associated with the electrode 212 and the electrode 213 is associated with the electrode 214. Then the current flows in the longitudinal direction between the associated rings and heats the variable stiffness material 201. There are provided temperature sensors, but they are not shown in the Fig. 4. Reference numeral 216 is the connecting wire for electrode 211, wires 215 connect the second and third electrode 212 and 213 with the outside and electrode 214 is connected with a further wire in the shaft material to the outside.

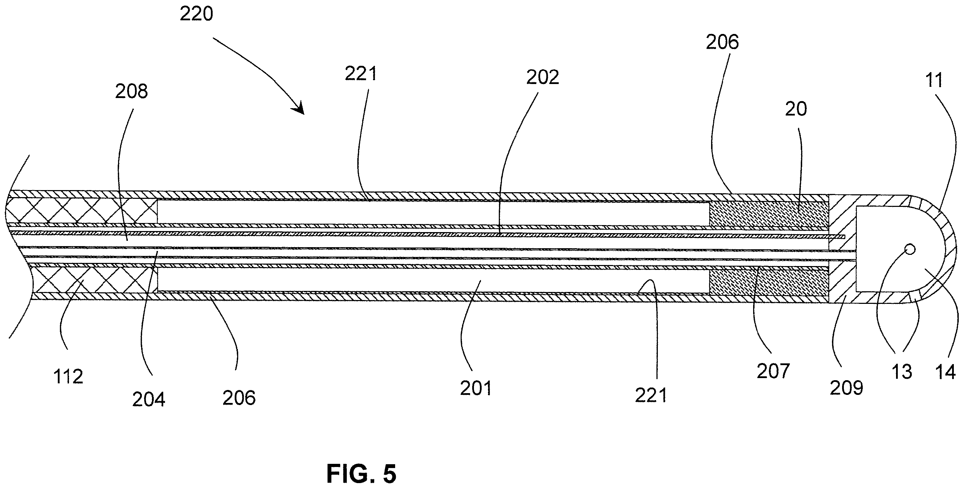

Fig. 5 shows a schematic cross-section side view of a variable stiffness section 220 according to a third embodiment of the invention using a deposited layer 221. Said cis provided as coating on the inner side of the outer flexible tube 207. The connection of the resistive layer 221 with the power supply and the control unit is not shown in Fig. 5.

In all embodiments, the plurality of variable stiffness sections 100, 105, 218, 218' can be separated by a heat isolating segment as element 215 to reduce the heat transfer between the variable stiffness sections.

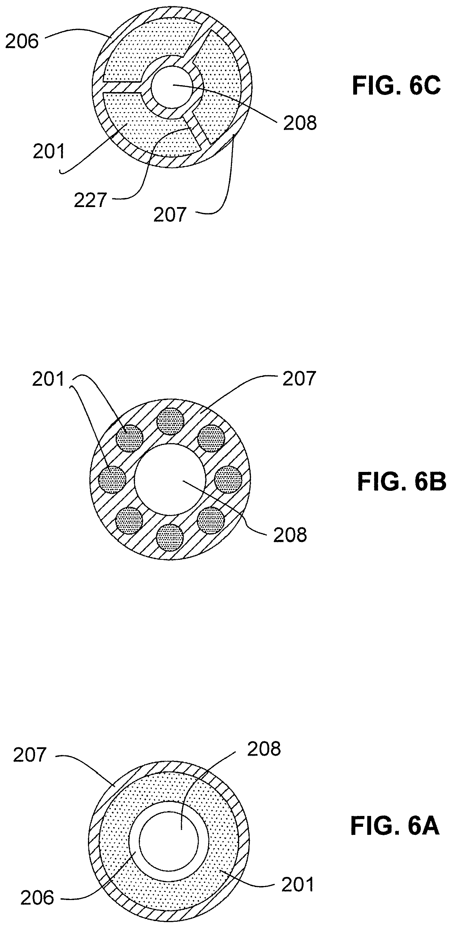

The arrangement of the catheter cross section can be adapted to the application requirements as shown in Fig. 6A, Fig. 6B and Fig. 6C, all showing a schematic cross-section of a catheter according to different embodiments of the invention.

Fig. 6A shows a cross section as applicable for Fig 3 to 5 (if an resistive coating would not be drawn in this Fig. and/or connecting wires are also not shown), with the variable stiffness material filling the ring of the cross-section along a specific length of the catheter.

Fig. 6B shows a almost solid flexible tube, combing outer 207 and inner 206 tube. This solid flexible tube has here eight longitudinal through bores to receive the variable stiffness material 201.

Fig. 6C shows an embodiment similar to Fig. 6A with three radial webs 227 separating the variable stiffness material 201 into three compartments. This allows to have multiple lumens filled with a variable stiffness material 201.

Additional lumens 208 and/or 204 can also be used as a channel for inserting a tool or wire for the catheter tip. Wires can also be integrated directly inside the variable stiffness material to provide power or signal transmission to the catheter tip. For example for radio frequency ablation we can integrate some wires for the ablation tip and one channel for delivering a fluid for ablation tip cooling which is expelled through the irrigation holes 13.

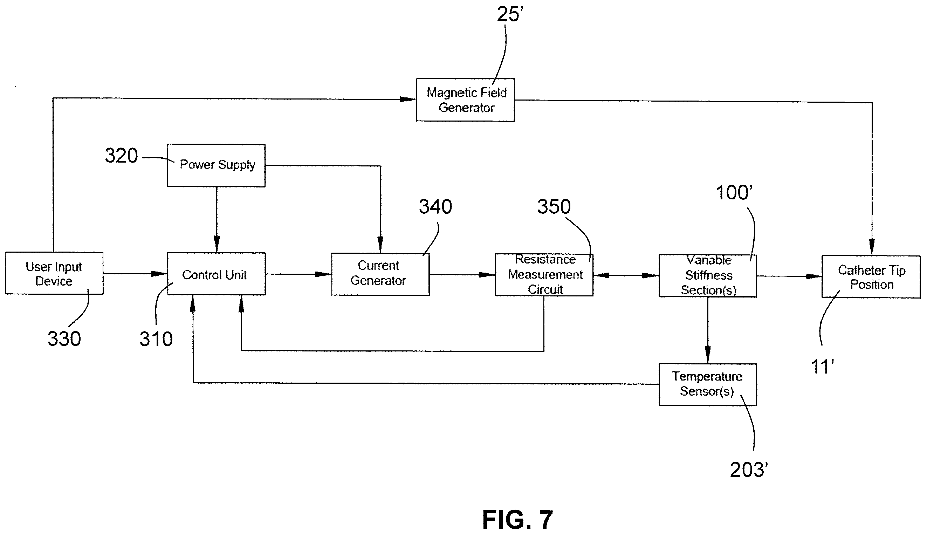

Fig. 7 shows a diagram of a control system for a catheter 10 according to an embodiment of the invention. The user input device 330 is connected and control the magnetic field generator 25' as well as the control unit 310 for the temperature control. The control unit 320 is also connected with the power supply 320 providing the necessary power to the current generator 340 delivering the heating current to the resistive portion at the variable stiffness section 100' via a resistance measurement circuit 350. The temperature of the variable stiffness material 201 is monitored by temperature sensors 203'. The catheter tip position 11' is influenced by the magnetic field direction 25/26 provided through the magnetic field generator 25'.

The temperature of the low melting point material can be monitored directly by measuring the electrical resistance in circuit 350 of the low melting point alloys or indirectly by measuring the resistance of the wire used for resistive heating. The change of resistance can then be fed to a control unit 310 that can keep the resistance around a set point and control the current flowing through the variable stiffness segment. The resistance corresponding to the phase change can be calibrated using resistance measurements during variable stiffness material cool down. The resistance measurements in the heating wire will show a plateau during the phase change as the temperature stays constant during phase changes. A simple implementation of a controller is to maintain the resistance slightly above the phase change resistance to keep the catheter in the flexible state or slightly lower to maintain it into the solid and rigid state. It is also possible to control it to any intermediate value to change continuously the stiffness of the catheter.

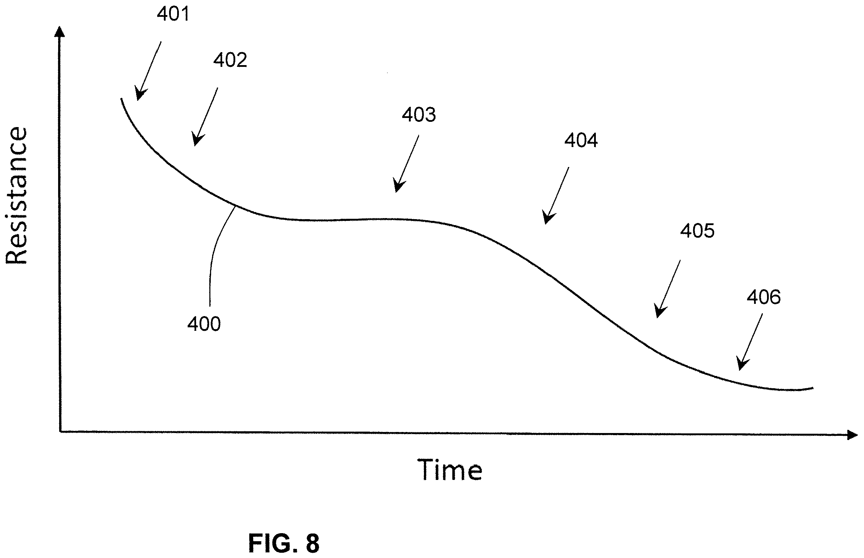

Fig. 8 shows a diagram of resistance against time of an example of a resistive wire resistance variation over time during cooling down. The curve 401 can be characterized as follows. The first section 401 relates to a quick decrease of resistance in the liquid state. The adjacent flattening section 402 is related to the resistance in the liquid state before phase change. Then curve 400 becomes a plateau section 403 with an almost constant resistance and temperature. Curve section 404 relates to a restart of resistance change in solid state after phase change. The next section 405 is related to quick resistance changes and temperature decay in the solid phase.

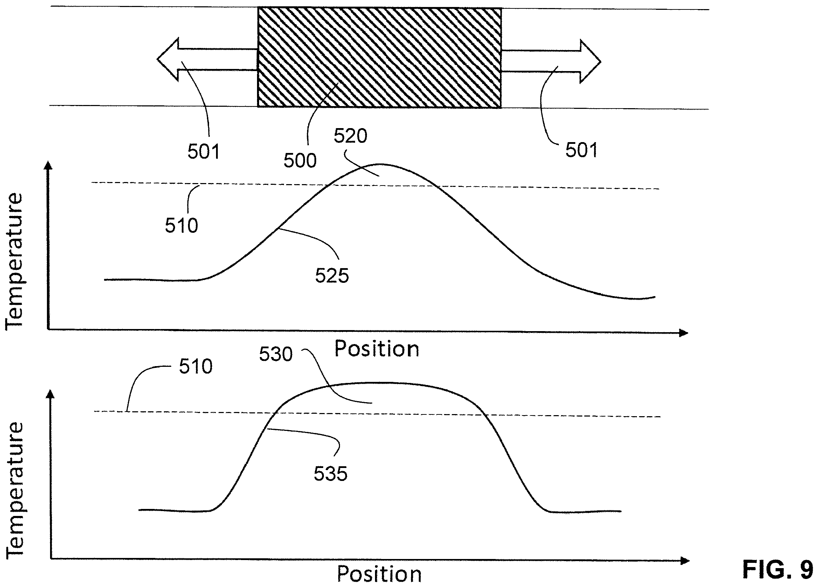

Fig. 9 shows a schematic view of the effect of a heated heat source 500 for a small or high energy injection. The heat source 500 dissipates heat in the directions 501 along the catheter. This heat source 500 is shown in the same scale as the two curves 520 and 530 for the temperature against the longitudinal position of the catheter.

The melting temperature 510 shows that less energy dissipation leads to a shorter section 520 with a temperature higher than the melting temperature 510, i.e. the length where the curve 525 reflects a higher temperature than the melting temperature 510.

The melting temperature 510 shows on the other side that higher energy dissipation leads to a longer section 530 with a temperature higher than the melting temperature 510, i.e. the length where the curve 535 reflects a higher temperature than the melting temperature 510.

Providing more heat as energy dissipates will increase the length of the melted segment by heat conduction or convection along the variable stiffness core. In the opposite providing less energy as dissipated will result in a decrease of the melted length of the segment. This results in a continuous change of stiffness relative to the energy injected.

Temperature control can also be implemented by integrating temperature sensors into the catheter. This temperature can be fed to the controller to regulate the current.

The variable stiffness material 201 can be an LMPA (low melting point alloy). Such an LMPA or fusible alloy are metal alloys that can be melted at relatively low temperature (in general below 183° Celsius). Those alloys are composed of a mixture of Bismuth, Lead, Tin, Indium, Cadmium, Thallium, and Gallium. Some of the most common low melting point alloys are commercially obtainable Rose's metal, Cerrosafe, Wood's metal, Cerrolow 136 and Cerrolow 117.

An alternative to low melting point alloys is to use a low melting point polymer as disclosed in H. Dong and G. M. Walker, "Adjustable stiffness tubes via thermal modulation of a low melting point polymer," Smart Mater. Struct., vol. 21, no. 4, p. 42001, Apr. 2012. It is also possible to use waxes as disclosed in N. G. Cheng, A. Gopinath, L. Wang, K. Iagnemma, and A. E. Hosoi, "Thermally Tunable, Self-Healing Composites for Soft Robotic Applications," Macromol. Mater. Eng., vol. 299, no. 11, pp. 1279-1284, Nov. 2014. Furthmore, any material with a large change of stiffness in a temperature range between 20°C and 150°C can be used as disclosed in M. Manti, V. Cacucciolo, and M. Cianchetti, "Stiffening in Soft Robotics: A Review of the State of the Art," IEEE Robot. Autom. Mag., vol. 23, no. 3, pp. 93-106, Sep. 2016.

The variable stiffness material and heating mechanism can be replaced by any stiffening mechanism or combination of them (i.e. fluidics, tendons, electro active polymers, SMA, braided sleeve, flexible layer, granular jamming, layer jamming, electro-magneto-rheological material, glass transition material, shape memory materials, conductive polymer or chemical based).

Depending of the design and the application the variable stiffness segment 100 etc. can have an external diameter between 0.5mm and 20mm. The length of the device can vary in function of the operating site going from 2mm (i.e. eye catheter) to long device 8m (i.e. small intestine). For a long device only the first 300mm need to have combined magnetic field steering and controlled of the stiffness.

In the flexible state the variable stiffness segment should be able to bend at less 45° with a magnetic field of 250mT.

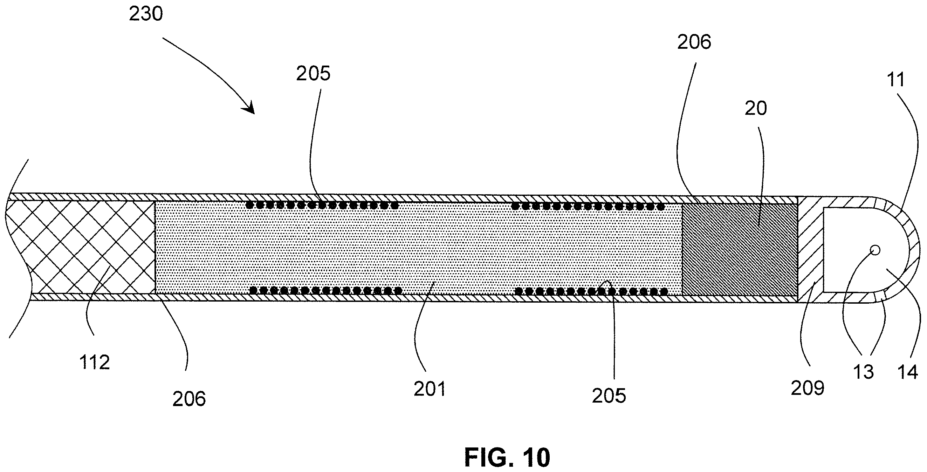

Fig. 10 shows a schematic cross-section side view of a variable stiffness section 230 according to a further embodiment of the invention wherein the flexible tube 206 is a single wall and the variable stiffness material 201 fills essentially the entire lumen of the variable stiffness section 230. The catheter tip 11 may comprise a cavity 14 and irrigation holes 13, but it is also possible that the tip comprises further elements as ablation electrode parts, since it is closed by the rear wall 209 which may comprise passages for electric connections. The magnet 20 is positioned against this rear wall 209 between the flexible tube wall 206. It is possible but not necessary that the cylindrical magnet 20 is separated from the variable stiffness section 230 by an additional wall. The variable stiffness section 230 ends with shaft material 112 filling the lumen between the flexible tube walls 206. Here, the variable stiffness material 201 is confined between the magnet 20 and the shaft material 112 filling essentially the entire volume of the lumen in between beside here four electrodes 205 attached at the inside of the tube wall 206. It is also possible that the electrodes 205 are provided centrally at a connecting structure between a cross-section oriented closing wall near the magnet 20 and the shaft material 112 or a similar wall there; but heating at the tube walls 206 is preferred. The current is then flowing directly inside the variable stiffness material 201 via these helical two electrodes 205. Of course, any number of electrodes 205 are possible and they do not need to be helical but can comprise e.g. longitudinal strips with connecting circumferential connections. Such electrodes 205 can also be used in the other embodiments of the invention. All features identical to features of the embodiment of Fig. 3 to 5 have the same reference numerals and are not mentioned again here. This is e.g. true for connecting wires for the electrodes 205 or wires leading through section 230 to the catheter tip 11. Fig. 10 can be considered to provide an embodiment with the least minimal elements that characterize the invention with shaft material 112 (or a closing cross-section oriented wall), a magnet 20 on the other longitudinal end of the variable stiffness portion 230, the variable stiffness material 201, at least one (here already two) resistive wires 205 in the variable stiffness material 201, here already provided on the inside of the outside flexible tubing 206. The two electrodes 205 allow for only activating sections of electrodes 205 (which could be at more than two sub sections) to liquefy the material "around", i.e. in the vicinity of the heating sections, i.e. there will be no clear cut-off as with radial separations.

**LIST OF REFERENCE SIGNS**

| | | | |
|---|---|---|---|
| 10 | catheter | 208 | lumen |
| 10' | catheter | 209 | rear wall |
| 10" | catheter | 210 | variable stiffness section with direct heating |
| 11 | catheter tip | | |
| 11' | catheter tip position | 211 | electrode |
| 12 | cathether shaft | 212 | electrode |
| 13 | irrigation hole | 213 | electrode |
| 14 | cavity | 214 | electrode |
| 15 | variable stiffness region | 215 | thermal isolation |
| 20 | magnet | 216 | wiring for electrode |
| 25 | direction of magnetic field | 217 | wiring for electrodes |
| 25' | magnet field generator | 218 | first variable stiffness section |
| 26 | direction of magnetic field | 218' | second variable stiffness section |
| 100 | variable stiffness section | | |
| 100' | variable stiffness section | 220 | variable stiffness section with deposited layer heating |
| 101 | proximal part | | |
| 105 | further variable stiffness section | 221 | resistive coating layer |
| | | 227 | radial web |
| 112 | shaft material | 230 | variable stiffness section with single wall tube |
| 200 | variable stiffness section with resistive wire heater | | |
| | | 310 | control unit |
| 201 | variable stiffness material | 320 | power supply |
| 202 | wire for tip | 330 | user input device |
| 203 | temperature sensor | 340 | current generator |
| 203' | temperature sensor | 350 | resistance measurement unit |
| 204 | lumen for cooling fluid and/or tool channel | 400 | curve |
| | | 401 | quick decrease of resistance |
| 205 | resistive wire | 402 | resistance before phase change |
| 206 | flexible tube encapsulation | | |
| 207 | flexible tube encapsulation | 500 | heat source |
| 501 | heat transfer direction | 525 | temperature against position |
| 510 | melting temperature | 530 | long melted section |
| 520 | short melted section | 535 | temperature against position |

## Claims

1. A steerable catheter (10) with portions (100, 105, 200, 210, 220, 230) of different stiffness, having: a proximal end (11), a distal end, a lumen (208) extending there between, at least one magnet (20) at the proximal end (11), at least a magnetic field (25, 26) to influence the proximal end (11), wherein portions (100, 105, 200, 210, 220) of the steerable catheter (10) can be of different stiffness, **characterized in that** the portions (100, 105, 200, 210, 220, 230) of different stiffness comprise at least one portion of variable stiffness based on a phase change between a liquid phase and a solid phase of a liquid-solid material (201) contained in a longitudinal section of the catheter providing a cavity for this liquid-solid material (201) and having additional control elements (205; 211, 212, 213, 214; 221) configured to influence the phase of the liquid-solid material (201).

2. The steerable catheter (10) according to claim 1, wherein the liquid-solid phase transition is based on heating or cooling off the liquid-solid material (201).

3. The steerable catheter (10) according to claim 2, wherein the control elements comprise a resistive wire heating coil (205).

4. The steerable catheter (10) according to any one of claims 1 to 3, wherein the different stiffness portions (230) comprise at least one longitudinal section with an outer flexible tube (206) and radially the cross-section of the different stiffness portions (230) closing walls, wherein the liquid-solid material (201) is provided between this flexible tube (206) and said longitudinal closing walls.

5. The steerable catheter (10) according to any one of claims 1 to 3, wherein the portions (100, 105, 200, 210, 220) comprise at least one double-walled longitudinal section with an outer flexible tube (206) and an inner flexible tube (207) and radially the cross-section of the different stiffness portions (100, 105, 200, 210, 220) closing walls, wherein the liquid-solid material (201) is provided between these mentioned flexible tubes (206, 207) and said longitudinal closing walls.

6. The steerable catheter (10) according to any one of claims 2 to 5, wherein the control elements comprise direct heating through electrodes (211, 212, 213, 214) provided across the cross-section of the catheter between at least two positions in the longitudinal direction axis.

7. The steerable catheter (10) according to any one of claims 2 to 5, wherein the control elements comprise direct heating through electrodes (205) provided across the catheter as helical electrodes provided between the inner side of the outer flexible tube (206) and, if at a double-walled section, the outer side of the inner flexible tube (207).

8. The steerable catheter (10) according to claim 7, wherein the helical electrodes (205) are provided attached at the inner side of the outer flexible tube (206).

9. The steerable catheter (10) according to any one of claims 2 to 8, wherein the liquid-solid phase transition is based on heating or cooling off the liquid-solid material (201) through peltier elements, provided in the walls (206), preferably in the inner walls of the cavity for the liquid-solid material.

10. The steerable catheter (10) according to any one of claims 1 to 9, wherein wiring for the electrodes (205) and/or wiring for a functional tip (11) of the catheter is provided within the at least one different stiffness portion (100, 105, 200, 210, 220, 230), especially as connecting wires provided in the longitudinal direction of the catheter.
